# EUROPEAN PATENT SPECIFICATION

(11) **EP 0 483 910 B1**
(45) Date of publication and mention of the grant of the patent: **06.12.1995**
(21) Application number: 91202730.7
(22) Date of filing: 22.10.1991
(51) Int. Cl.: G01N 33/543, B01L 3/00, G01N 33/569

(54) **Batch test apparatus for immunoassay**
Mehrfach-Nachweisvorrichtung
Dispositif d'essai multiple simultané

(30) Priority: 29.10.1990 US 604285
(43) Date of publication of application: 06.05.1992
(73) Proprietor: Johnson & Johnson Clinical Diagnostics, Inc., Rochester New York 14650 (US)
(72) Inventor: Babaoglu, Kerinchan, c/o EASTMAN KODAK Co., Rochester, NY 14650-2201 (US); Norkus, Norbert S., c/o EASTMAN KODAK Co., Rochester, NY 14650-2201 (US); Johnson, Randall S., c/o EASTMAN KODAK Co., Rochester, NY 14650-2201 (US)
(74) Representative: Mercer, Christopher Paul

(56) References cited:
- EP-A- 0 128 422
- EP-A- 0 177 352
- EP-A- 0 308 231
- WO-A-87/03218
- DE-U- 9 015 317
- US-A- 4 245 043

## Description

This invention relates to batch test apparatus, and is more particularly concerned with such apparatus, a kit, and method for doing immunoassays, which use positive and negative controls and a visual comparison of the patient sample result against the result of the negative control.

Many test devices have been provided for conducting immunoassays. In some instances the device is personalized to test only one patient sample, a positive control and a negative control, using three test wells as described in US-A-4 847 199 and US-A-4 921 677.

In such devices, the negative control serves two functions, namely, to show what background color, if any, can be expected without the targeted antigen being present, and to act as a control in the event the reagents should become damaged, for example, denatured to the point that any sample will cause a color to form whether or not the targeted antigen is present. As is well known, such devices are read by visually comparing color changes. Such devices are limited in that multiple patient testing is not possible in any one device.

Other test apparatus has provided for batch testing of many samples, as well as for a positive control and a negative control. Examples are described in US-A-4 245 043 and EP-A-0 177 352, wherein test wells are arranged in rows and columns, and a row for example will be dedicated to being all a positive control, all a negative control, or a batch of patient samples. Thus, in the apparatus of US-A-4 245 043, rows A and K are strictly for negative controls, that is, produce no substantial color change, rows B and J are for positive controls and produce a substantial color change, and the intermediate rows C to I are for test samples. Similarly, in EP-A-0 177 352, one column is reserved for the "blank" (a negative control producing substantially no color change) whereas another column for the positive control (labeled "standard"). All other columns of the apparatus are used for samples.

It will be apparent that the batch testing apparatus described in US-A-4 245 043 the row or column of test wells which produces no substantial color change has interposed between it and the rows or columns of test sample wells, a row or column, respectively, which always produces a substantial color change (the positive control). This arrangement has a substantial disadvantage in that the user is unable to visually compare a negative control test well (mostly colorless) directly with a test sample well, without having to look at an intermediate well between them which has a substantial color change. This is particularly troublesome when a patient sample well produces a color change which is only slightly more than the negative well, but not nearly as much as the positive well positioned between it and the negative well. In such a case, the positive well color change is so great as to make difficult, if not impossible, a determination as to whether the patient sample is in fact enough greater in color change than the negative control to count as a "positive". This problem is further compounded, as in the case described in EP-A-0 177 352 noted above, when test sample wells farthest away from the negative control well, have to be compared while visually skipping over the other test sample wells in the same row which may have a greater color change than the one in question. Even if not all of the intervening test wells are used, the mere fact of their location between the wells to be examined creates difficulties.

All these problems can lead to substantial error in estimating if a particular test sample well is in fact a "positive" rather than a "negative" result.

It is therefore an object of the present invention to provide a batch testing apparatus wherein the patient or test sample wells are all easily and accurately contrasted by eye with the negative wells to determine if the results are not negative.

More particularly, in accordance with one aspect of the present invention, there is provided test apparatus for conducting immunoassays comprising a plurality of test wells each constructed to receive a liquid and each being positioned to form at least two rows with at least two of the wells in each row, at least some of the wells being constructed and designated to receive a negative control liquid and the remainder of the wells being constructed and designated to receive either a positive control liquid or a patient sample liquid;
characterized in that the at least some wells form a portion of a first row and the remainder wells form a portion of a second row located adjacent to the portion of the first row with no well from the remainder wells being positioned in between the first row and the second row thereby providing a readily available comparison between wells in the first row and wells in the second row.

In accordance with a second aspect of the present invention, there is provided a test kit for conducting immunoassays comprising a frame and a plurality of housings removably mounted in the frame, each of the housings having a plurality of test wells positioned to form at least two rows with at least two of the wells in each row, the wells of one of the rows being constructed and designated to receive only materials for negative control and the wells of the other row being constructed as color-producing wells which are designated to receive only either materials for positive control or patient sample such that no color-producing well is positioned between a well for patient sample liquid and a negative control well.

In accordance with a third aspect of the present invention, there is provided a method for conducting an immunoassay for a targeted antigen or antibody in a plurality of patient samples using a plurality of test wells and a positive and negative control, the controls comprising the reagents which, if properly combined and maintained in active form, will always form a substantial color change in the case of the positive control and never form a substantial color change in the case of the negative control in the absence of the sought-for antigen or antibody, the method comprising the steps of:-
a) arranging the test wells into two rows, each well in a row being adjacent to a well of the other row;
b) depositing into the wells of one of the rows only the materials of the negative control in the proper sequence which produces no substantial color change;
c) depositing into the respective wells of the other of the rows either the patient samples or some of a positive control, and the reagents necessary to test for a targeted antigen or antibody; and
d) comparing the lack of a substantial color change in the wells of the one row with the presence or absence of a substantial color change in a well of the other row adjacent thereto, to determine that the targeted antigen or antibody is present or absent in the patient sample wells.

Accordingly, it is an advantageous feature of the invention that a batch test apparatus is provided wherein a test sample well is readily and accurately visually compared against a negative control well, because there is no well between them having a substantial color change.

It is another advantageous feature of the invention that such test apparatus can be constructed of variable length as needed.

For a better understanding of the present invention, reference will now be made, by way of example only, to the accompanying drawings in which:-
Figure 1 is an exploded perspective view of a test apparatus constructed in accordance with the present invention;
Figure 2 is a frame for mounting the housings which provide the test apparatus as shown in Figure 1;
Figures 3 and 4 are respective plan views of the apparatus shown in Figure 1 illustrating its use;
Figure 5 is a plan view similar to that shown in Figure 3, but of a second embodiment; and
Figure 6 is a plan view similar to that shown in Figure 4, but illustrating a third embodiment.

The invention is hereinafter described by reference to plural housings, each of which has only three test wells, which together provide two rows of nine wells each, to test for chlamydia using one of two preferred immunoassays. In addition, the invention is useful regardless of how many wells is provided in a single housing, of how many wells is provided in a single row, and regardless of the antigen being tested or the protocol used.

As noted, the assay is preferably for chlamydia, which as is conventional requires the extraction of the antigen from a patient's genital/urinary tract sample. This is done by conventional extraction techniques, the details of which have been omitted here. Two alternate immunoassay techniques are used after the antigen is deposited on to a suitable filter membrane.

In one technique, a monoclonal antibody (MAb) specific to the chlamydia antigen is added to the membrane, where it complexes with the antigen, if present, and otherwise passes through the membrane. Thereafter, a labeled antibody to that antibody is added which will complex with the first MAb (if present), and otherwise it will pass through the membrane.

In the other, and more preferred, technique, the MAb is preincorporated with a label, so that no second antibody need be added.

In either case, the label is preferably horse radish peroxidase (HRP). Thereafter (in either case), a dye solution is added, which contains at least a leuco dye and hydrogen peroxide as is conventional. In the presence of the HRP, the hydrogen peroxide will convert the leuco dye into dye, the concentration and color of which is proportional to the amount of antigen present.

The assay uses positive and negative controls. As is conventional, the positive control is a pre-prepared solution of chemically inactivated chlamydia elementary bodies which will complex with the MAb noted above. The negative control is a MAb or polyclonal antibody which is specific to a "nonsense" antigen, that is, an antigen that should never appear, for example, a site-specific protein not normally produced nor found in the sample or sampling site. All controls and antibody solutions include preservatives and/or surfactants, where appropriate (and as is conventional).

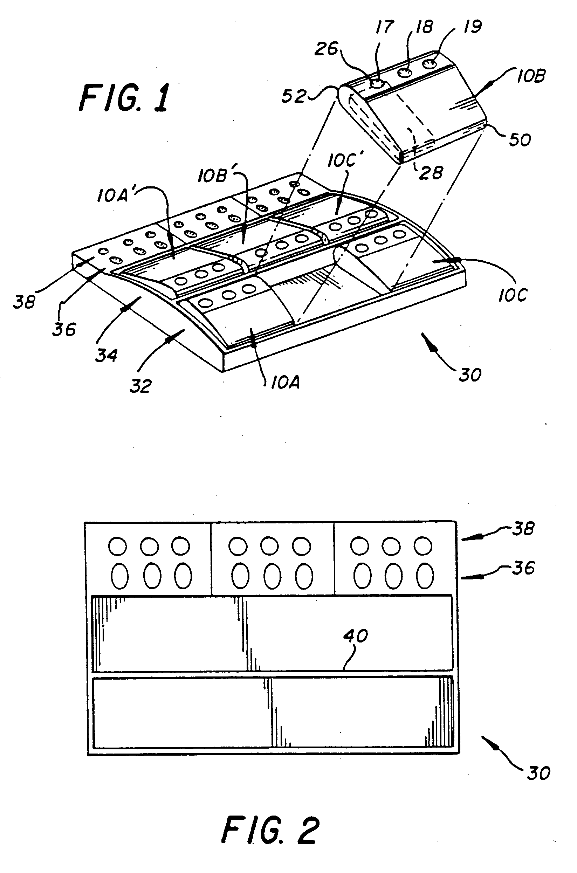

As shown in Figure 1, and in accordance with one aspect of the invention, the test wells comprise wells 17, 18 and 19 in a row in housings 10A, 10B, 10C, and 10A′, 10B′, and 10C′. Each of the housings in turn is mounted in a frame 30, so that a row 32 of three housings 10A, 10B and 10C is formed adjacent and generally parallel to a row 34 of three housings 10A′, 10B′ and 10C′. Rows 32 and 34 are formed by positioning the housings back to back. Each of these rows then provides a row of wells, numbered hereinafter as 32 and 34. Frame 30 can also optionally include two rows of apertures 36 and 38, each row for holding a tube of already-extracted patient sample (not shown) and a swab tube holder (not shown) respectively. The side walls of apertures 36 and 38 are sloped inward to hold the tubes and tube holders, as shown in Figure 1, or they can be vertical. A divider 40, as shown in Figure 2, is preferably included to separate the two rows 32 and 34. The swab holders, extraction tubes, frame 30, reagent bottles (not shown) and all the housings provide a convenient test kit.

Because each housing 10A, etc. has only three wells, the number of wells provided in either row can be any multiple of three - for example, nine as shown, or six or three. In fact, by making frame 30 of any appropriate length, it can be any multiple of three.

Any desired construction can be given to housings 10A, 10A′, and so forth. Preferably it is that described in US-A-4 847 199 and US-A-4 921 677.

The filter membrane (not shown) in such housings is located at the bottom of sloping walls 26, and underneath each such membrane is positioned a liquid-absorbing material 28 (shown in phantom in Figure 1). A sliding vent bar 50 can be mounted on the front, and back portion 52 is preferably rounded. (For simplicity, only housing 10B illustrates the vent bar 50 and the sloping walls 26 in wells 17 to 19.)

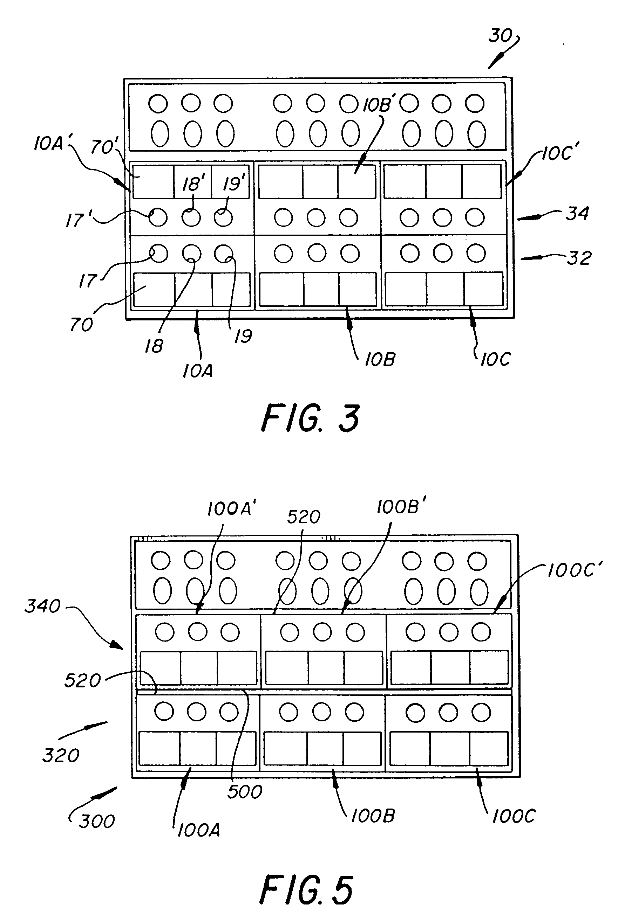

In accordance with another aspect of the invention, rows 32 and 34 of the housings are used as follows (Figure 3): In the wells of row 34, preferably only the materials for negative control are deposited during the protocol. In the wells of row 32, only the potentially color-producing materials are placed, that is, either the patient sample (in most cases) or the positive control (in one well only). Because the positive control may be run only once in a day, any given set of nine wells in row 32 will probably only have patient (test) sample liquid. Importantly, this arrangement means that no well will be positioned between a well of row 32 and its adjacent negative control well of adjacent row 34. Thus, there will be nothing to prevent an accurate comparison of the color (if any) produced by a patient sample in a well of row 32, with the minimal or no color produced in negative control wells of row 34. Most importantly, no positive control well nor a patient sample well appears between a well of row 32 and a well of row 34. If a positive control well is run at all, it will be one of the wells in row 32.

The following then is a preferred protocol for using the wells of rows 32 and 34, having already extracted whatever chlamydia antigen may be present in the patient samples:
a) In a pair of wells, e.g., 17, 17′ of all the housings, five drops of the patient sample (or the positive control, if that is to be used at this time) is placed in each well. The appropriate patient identification, or symbol "+", respectively (if the positive control is being tested), is placed on label portions 70 and 70′.
b) Each of the paired wells is treated with a different solution of different patient sample.
c) The sample solutions are allowed to drain through the filter membrane.
d) A wash solution is added rapidly and turbulently to fill about two-thirds of each well, and it is allowed to drain.
e) Two drops of the reagent comprising the negative control solution noted above are added to all the wells of row 34 only.
f) Two drops of the reagent comprising the labeled MAb specific to chlamydia are added to all the wells of row 32 only.
g) All rows are allowed to incubate for two minutes.
h) A turbulent wash is added to all the wells of both rows.
i) Two drops of the reagent comprising the dye solution noted above are added to all the wells of both rows 32 and 34.
j) The wells are allowed to incubate for 3 minutes.

The wells are now ready to be read, by visually comparing the presence or absence of color produced in row 32 against the minimal (if any) color of the negative control wells of row 34. Presence of substantially more color in a well of row 32 than is present in a well of row 34 means chlamydia is present in the sample tested. (As used herein, "substantial" means an amount which is clearly distinguishable from that produced by the negative control well.)

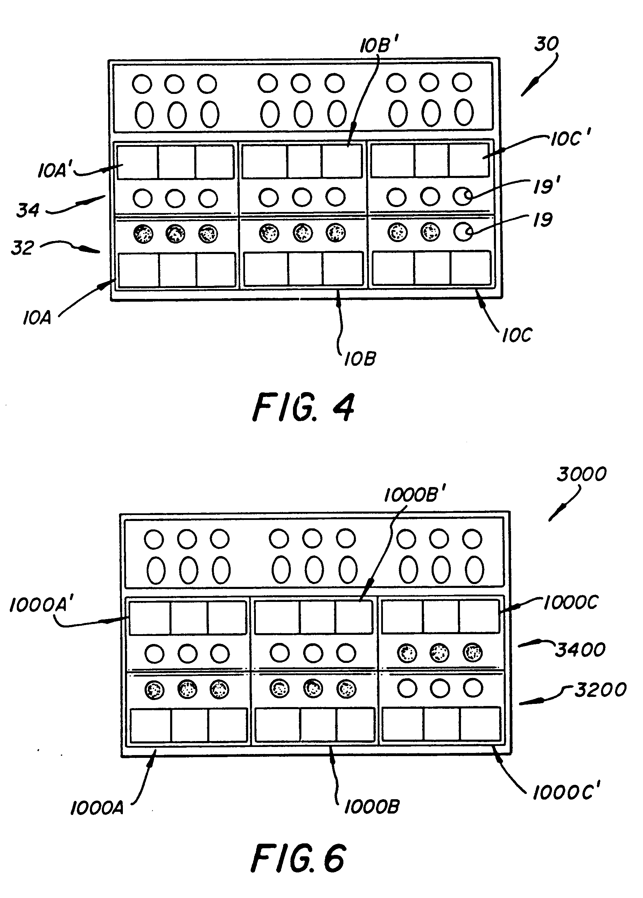

As a result, the color produced by the patient sample wells of row 32, shown as hatch lines in Figure 4, is easily contrasted with the lack of color in the negative control wells of row 34. If a well such as well 19 of housing 10C is as colorless as the adjacent well 19′ of housing 10C′, the test is negative for the sample in well 19.

It will be readily appreciated that the above-described test apparatus and protocol is equally useful to test for an antibody, for example, the HIV antibody. In such a case, the membrane need only be treated to cause the targeted antibody to attach, as the first sequence of steps a) to d) described above. The label that is then added can be, e.g., a labeled antibody to that antibody, as is well known.

It is not essential that the housings of rows 32 and 34 be disposed back to back, as shown. Alternatively, as shown in Figure 5, they can be placed in frame 30 so that they are back to front. Parts similar to those previously described bear the same reference numeral to which the distinguishing digit "0" is added. Thus, frame 300 has in it a row 320 of housings 100A, 100B and 100C, and a row 340 of housings 100A′, 100B′ and 100C′. Each housing is constructed and processed identically as described for the previous embodiment. The only difference is that housing 100A′ has its front portion 500 adjacent to back portion 520 of housing 100A, and so forth for the remaining pairs of housings.

Since there still is no intermediate well disposed between the wells of row 320 and the wells or row 340, such an arrangement also produces an accurate and easy determination of positive or negative patient samples.

As yet another alternative, it is not essential that the negative control wells all occupy only, for example, row 34. Instead, as shown in Figure 6, one pair of housings can be switched. Parts similar to those previously described bear the same reference numerals to which the distinguishing digits "00" have been added. Thus, some of the wells of row 3200, those provided by housing 1000C′, are negative controls producing no substantial color to be compared with substantially colored wells of housing 1000C in row 3400. (The remainder of row 3400, as provided by housings 1000A′ and 1000B′, would still be negative controls.) This works, because the patient sample test wells of housing 1000C, now in row 3400, are still adjacent a substantially non-colored well with no intervening color-producing well to confuse the readings. The color-producing wells have been hatched in Figure 6 to render clear the alternate arrangement achieved by switching the positions of housings 1000C and 1000C′. However, such switching as shown in Figure 6 is not as preferred as the alignment shown in, for example in Figure 4, because the operator could become confused as to which wells should contain the negative control, and so forth.

As a further alternative, not shown, all the wells of a given row, for example, row 32 or 34, can be provided in a single housing instead of a plurality of housings. Still further, all of the housings for both rows can be integrated into a single device.

## Claims

1. Test apparatus (10A, 10B, 10C, 10A′, 10B′, 10C′, 30; 100A, 100B, 100C, 100A′, 100B′, 100C′, 300; 1000A, 1000B, 1000C, 1000A′, 1000B′, 1000C′, 3000) for conducting immunoassays comprising a plurality of test wells (17, 18, 19, 17′, 18, 19′) each constructed to receive a liquid and each being positioned to form at least two rows (32, 34; 320, 340; 3200, 3400) with at least two of the wells (17, 18, 19, 17′, 18′, 19′) in each row (32, 34; 320, 340; 3200, 3400), at least some of the wells (17′, 18′, 19′) being constructed and designated to receive a negative control liquid and the remainder of the wells (17, 18, 19) being constructed and designated to receive either a positive control liquid or a patient sample liquid;
characterized in that the at least some wells (17, 18, 19) form a portion of a first row (32; 320; 3200) and the remainder wells (17′, 18′, 19′) form a portion of a second row (34; 340; 3400) located adjacent to the portion of the first row (32; 320; 3200) with no well from the remainder wells (17′, 18′, 19′) being positioned in between the first row (32; 320; 3200) and the second row (34; 340; 3400) thereby providing a readily available comparison between wells (17, 18, 19) in the first row (32; 320; 3200) and wells (17′, 18′, 19′) in the second row (34; 340; 3400).

2. Apparatus according to claim 1, wherein the wells (17, 18, 19, 17′, 18′, 19′) of each row (32, 34; 320, 340; 3200, 3400) are formed in plural removable housings (10A, 10B, 10C, 10A′, 10B′, 10C′; 100A, 100B, 100C, 100A′, 100B′, 100C′; 1000A, 1000B, 1000C, 1000A′, 1000B′, 1000C′) so that the rows (32, 34; 320, 340; 3200, 3400) can be varied in length.

3. Test apparatus (10A, 10B, 10C, 10A′, 10B′, 10C′, 30; 100A, 100B, 100C, 100A′, 100B′, 100C′, 300; 1000A, 1000B, 1000C, 1000A′, 1000B′ 1000C′, 3000) for conducting immunoassays comprising a plurality of test wells (17, 18, 19, 17′, 18′, 19′) each with a bottom surface constructed to receive any appropriate antigen or antibody that may be present in a sample and to form an antigen-antibody complex,
characterized in that the wells (17, 18, 19, 17′, 18′, 19′) are mounted in at least two generally adjacent and parallel rows (32, 34; 320, 340; 3200, 3400), one row (34; 340; 3400) being constructed to receive only those materials which will produce no visible color change due to the sought-for antigen or antibody, as a negative control, and the row (32; 320; 3200) adjacent the one row (34; 340; 3400) being constructed to receive only those assays which can produce a color change, as either a patient sample containing the suspected antigen or antibody, or as a positive control containing an antigen or antibody which will produce a color change in order to readily identify a patient sample which has the antigen or antibody by comparing each color-bearing well (17, 18, 19) of one row (32; 320; 3200) with a non-color-bearing well (17′, 18′, 19′) of the adjacent row (34; 340; 3400).

4. A test kit (10A, 10B, 10C, 10A′, 10B′, 10C′, 30; 100A, 100B, 100C, 100A′, 100B′ 100C′, 300; 1000A, 1000B, 1000C, 1000A′, 1000B′, 1000C′, 3000) for conducting immunoassays comprising a frame (30; 300; 3000) and a plurality of housings (10A, 10B, 10C, 10A′, 10B′, 10C′; 100A, 100B, 100C, 100A′, 100B′, 100C′; 1000A, 1000B, 1000C, 1000A′, 1000B′, 1000C′) removably mounted in the frame (30; 300; 3000), each of the housings (10A, 10B, 10C, 10A′, 10B′, 10C′; 100A, 100B, 100C, 100A′, 100B′, 100C′; 1000A, 1000B, 1000C, 1000A′, 1000B′, 1000C′) having a plurality of test wells (17, 18, 19; 17′, 18′, 19′) positioned to form at least two rows (32, 34; 320, 340; 3200, 3400) with at least two of the wells (17, 18, 19, 17′, 18′, 19′) in each row (32, 34; 320, 340; 3200, 3400), the wells (17′, 18′, 19′) of one of the rows (34; 340; 3400) being constructed and designated to receive only materials for negative control and the wells (17, 18, 19) of the other row (32; 320; 3200) being constructed as color-producing wells which are designated to receive only either materials for positive control or patient sample such that no color-producing well is positioned between a well (17, 18, 19) for patient sample liquid and a negative control well (17′, 18′, 19′).

5. A method for conducting an immunoassay for a targeted antigen or antibody in a plurality of patient samples using a plurality of test wells (17, 18, 19, 17′, 18′, 19′) and a positive and negative control, the controls comprising the reagents which, if properly combined and maintained in active form, will always form a substantial color change in the case of the positive control and never form a substantial color change in the case of the negative control in the absence of the sought-for antigen or antibody, the method comprising the steps of:-
a) arranging the test wells (17, 18, 19, 17′, 18′, 19′) into two rows (32, 34; 320, 340; 3200, 3400), each well (17, 18, 19, 17′, 18′, 19′) in a row being adjacent to a well of the other row;
b) depositing into the wells (17′, 18′, 19′) of one of the rows (34; 340; 3400) only the materials of the negative control in the proper sequence which produces no substantial color change;
c) depositing into the respective wells (17, 18, 19) of the other of the rows (32; 320; 3200) either the patient samples or some of a positive control, and the reagents necessary to test for a targeted antigen or antibody; and
d) comparing the lack of a substantial color change in the wells (17′, 18′, 19′) of the one row (34; 340; 3400) with the presence or absence of a substantial color change in a well (17, 18, 19) of the other row (32; 320; 3200) adjacent thereto, to determine that the targeted antigen or antibody is present or absent in the patient sample wells (17, 18, 19).

## Patentansprüche

1. Testvorrichtung (10A, 10B, 10C, 10A′, 10B′, 10C′, 30; 100A, 100B, 100C, 100A′, 100B′, 100C′, 300; 1000A, 1000B, 1000C, 1000A′, 1000B′, 1000C′, 3000) zum Durchführen von Immununtersuchungen, mit einer Anzahl von Testbehältern (17, 18, 19, 17′, 18′, 19′), die jeweils dafür vorgesehen sind, eine Flüssigkeit aufzunehmen und die so angeordnet sind, daß wenigstens zwei Reihen (32, 34; 320, 340; 3200, 3400) mit wenigstens zwei Behältern (17, 18, 19, 17′, 18′, 19′) in jeder Reihe (32, 34; 320, 340; 3200, 3400) gebildet werden, wobei wenigstens einige der Behälter (17′, 18′, 19′) dafür konstruiert und vorgesehen sind, eine negative Kontrollflüssigkeit aufzunehmen, und wobei die übrigen Behälter (17, 18, 19) dafür konstruiert und vorgesehen sind, entweder eine positive Kontrollflüssigkeit oder eine Patientenprobenflüssigkeit aufzunehmen;
dadurch **gekennzeichnet**, daß die wenigstens einigen Behälter (17, 18, 19) einen Teil einer ersten Reihe (32; 320; 3200) und die übrigen Behälter (17′, 18′, 19′) einen Teil einer zweiten Reihe (34; 340; 3400) bilden, der angrenzend an den Teil der ersten Reihe (32; 320; 3200) angeordnet ist, wobei sich keiner der übrigen Behälter (17′, 18′, 19′) zwischen der ersten Reihe (32; 320; 3200) und der zweiten Reihe (34; 340; 3400) befindet und so ein sofortiger Vergleich zwischen den Behältern (17, 18, 19) in der ersten Reihe (32; 320; 3200) und den Behältern (17′, 18′, 19′) in der zweiten Reihe (34; 340; 3400) möglich ist.

2. Vorrichtung nach Anspruch 1, wobei die Behälter (17, 18, 19, 17′, 18′, 19′) einer jeder Reihe (32, 34; 320, 340; 3200, 3400) in einer Anzahl von entnehmbaren Gehäusen (10A, 10B, 10C, 10A′, 10B′, 10C′; 100A, 100B, 100C, 100A′, 100B′, 100C′; 1000A, 1000B, 1000C, 1000A′, 1000B′, 1000C′) ausgebildet sind, so daß die Länge der Reihen (32, 34; 320, 340; 3200, 3400) variiert werden kann.

3. Testvorrichtung (10A, 10B, 10C, 10A′, 10B′, 10C′, 30; 100A, 100B, 100C, 100A′, 100B′, 100C′, 300; 1000A, 1000B, 1000C, 1000A′, 1000B′, 1000C′, 3000) zum Durchführen von Immununtersuchungen, mit einer Anzahl von Testbehältern (17, 18, 19, 17′, 18′, 19′) mit einer Bodenfläche, die dafür vorgesehen ist, ein geeignetes Antigen oder einen geeigneten Antikörper aufzunehmen, der in einer Probe vorhanden sein kann, und einen Antigen-Antikörper-Komplex zu bilden,
dadurch **gekennzeichnet**, daß die Behälter (17, 18, 19, 17′, 18′, 19′) wenigstens in zwei im wesentlichen benachbarten und parallelen Reihen (32, 34; 320, 340; 3200, 3400) angeordnet sind, wobei eine Reihe (34; 340; 3400) dafür vorgesehen ist, nur solche Materialien aufzunehmen, die als negative Kontrollprobe aufgrund des gesuchten Antigens oder Antikörpers keine sichtbare Farbänderung hervorrufen, und wobei die an die eine Reihe (34; 340; 3400) angrenzende Reihe (32; 320; 3200) dafür vorgesehen ist, nur solche Proben aufzunehmen, die entweder als Patientenprobe mit dem erwarteten Antigen oder Antikörper oder als positive Kontrollprobe mit einem Antigen oder Antikörper, das bzw. der eine Farbänderung hervorruft, eine Farbänderung hervorrufen können, um durch Vergleichen eines Farbe zeigenden Behälters (17, 18, 19) einer Reihe (32; 320; 3200) mit einem keine Farbe zeigenden Behälter (17′, 18′, 19′) in der angrenzenden Reihe (34; 340; 3400) leicht eine Patientenprobe identifizieren zu können, die das Antigen oder den Antikörper enthält.

4. Testausrüstung (10A, 10B, 10C, 10A′, 10B′, 10C′, 30; 100A, 100B, 100C, 100A′, 100B′, 100C′, 300; 1000A, 1000B, 1000C, 1000A′, 1000B′, 1000C′, 3000) zum Durchführen von Immununtersuchungen, mit einem Kasten (30; 300; 3000) und einer Anzahl von Gehäusen (10A, 10B, 10C, 10A′, 10B′, 10C′, 100A, 100B, 100C, 100A′, 100B′, 100C′; 1000A, 1000B, 1000C, 1000A′, 1000B′, 1000C′), die entnehmbar im Kasten (30; 300; 3000) angeordnet sind, wobei jedes der Gehäuse (10A, 10B, 10C, 10A′, 10B′, 10C′; 100A, 100B, 100C, 100A′, 100B′, 100C′; 1000A, 1000B, 1000C, 1000A′, 1000B′, 1000C′) eine Anzahl von Testbehältern (17, 18, 19; 17′, 18′, 19′) aufweist, die so angeordnet sind, daß wenigstens zwei Reihen (32, 34; 320, 340; 3200, 3400) mit wenigstens zwei Behältern (17, 18, 19, 17′, 18′, 19′) in jeder Reihe (32, 34; 320, 340; 3200, 3400) gebildet werden, wobei die Behälter (17′, 18′, 19′) einer der Reihen (34; 340; 3400) dafür konstruiert und vorgesehen sind, nur Materialien für eine negative Kontrollprobe aufzunehmen, und wobei die Behälter (17, 18, 19) der anderen Reihe (32; 320; 3200) als farberzeugende Behälter konstruiert sind, die dafür vorgesehen sind, nur entweder Materialien für eine positive Kontrollprobe oder eine Patientenprobe aufzunehmen, so daß sich zwischen einem Behälter (17, 18, 19) für eine Patientenprobenflüssigkeit und einem Behälter (17′, 18′, 19′) für die negative Kontrollprobe kein farberzeugender Behälter befindet.

5. Verfahren zum Durchführen von Immununtersuchungen auf ein bestimmtes Antigen oder einen bestimmten Antikörper in einer Anzahl von Patientenproben, mit der Verwendung einer Anzahl von Testbehältern (17, 18, 19, 17′, 18′, 19′) und einer positiven und einer negativen Kontrollprobe, wobei die Kontrollproben die Reagenzien umfassen, die, wenn sie richtig kombiniert und in der aktiven Form gehalten werden, bei der positiven Kontrollprobe immer eine wesentliche Farbänderung hervorrufen, während sie bei der negativen Kontrollprobe bei Abwesenheit des gesuchten Antigens oder Antikörpers nie eine wesentliche Farbänderung hervorrufen, wobei das Verfahren die Schritte
a) des Anordnens der Testbehälter (17, 18, 19, 17′, 18′, 19′) in zwei Reihen (32, 34; 320, 340; 3200, 3400), wobei jeder Behälter (17, 18, 19, 17′, 18′, 19′) in einer Reihe einem Behälter in der anderen Reihe gegenüberliegt;
b) des Beschickens der Behälter (17′, 18′, 19′) einer der Reihen (34; 340; 3400) nur mit Materialien für die negative Kontrollprobe in der richtigen Reihenfolge, so daß im wesentlichen keine Farbänderung hervorgerufen wird;
c) des Beschickens der jeweiligen Behälter (17, 18, 19) der anderen Reihe (32; 320; 3200) entweder mit Patientenproben oder einige mit einer positiven Kontrollprobe und den Reagenzien, die für den Test auf ein bestimmtes Antigen oder einen bestimmten Antikörper erforderlich sind; und
d) des Vergleichens des Fehlens einer wesentlichen Farbänderung in den Behältern (17′, 18′, 19′) der einen Reihe (34; 340; 3400) mit dem Vorhandensein oder dem Nichtvorhandensein einer wesentlichen Farbänderung in einem Behälter (17, 18, 19) in der daran angrenzenden, anderen Reihe (32; 320; 3200) umfaßt, um festzustellen, ob das bestimmte Antigen oder der bestimmte Antikörper in den Patientenprobenbehältern (17, 18, 19) vorhanden ist oder nicht.

## Revendications

1. Appareil de test (10A, 10B, 10C, 10A′, 10B′, 10C′, 30; 100A, 100B, 100C, 100A′, 100B′, 100C′, 300; 1000A, 1000B, 1000C, 1000A′, 1000B′, 1000C′, 3000) pour conduire des immunoessais comprenant plusieurs puits de test (17, 18, 19, 17′, 18′, 19′) construits chacun pour recevoir un liquide et chacun étant positionné pour former au moins deux rangées (32, 34; 320, 340; 3200, 3400) avec au moins deux des puits (17, 18, 19, 17′, 18′, 19′) dans chaque rangée (32, 34; 320, 340; 3200, 3400), au moins certains des puits (17′, 18′, 19′) étant construits et conçus pour recevoir un liquide témoin négatif et le reste des puits (17, 18, 19) étant construit et conçu pour recevoir soit un liquide témoin positif, soit un liquide échantillon de malade;
caractérisé en ce que les puits, au moins au nombre de quelques uns (17, 18, 19) forment une partie d'une première rangée (32; 320; 320) et le reste des puits (17′, 18′, 19′) forme une partie de la seconde rangée (34; 340; 3400) située de façon adjacente à la partie de la première rangée (32; 320; 3200) avec aucun puits des puits restants (17′, 18′, 19′) n'étant positionné entre la première rangée (32; 320; 3200) et la seconde rangée (34; 340; 3400) fournissant ainsi une comparaison facilement disponible entre les puits 17, 18, 19) dans la première rangée (32; 320; 3200) et les puits (17′, 18′, 19′) dans la seconde rangée (34; 340; 3400).

2. Appareil selon la revendication 1, dans lequel les puits (17, 18, 19, 17′, 18′, 19′) de chaque rangée (32, 34; 320, 340; 3200, 3400) sont formés dans plusieurs réceptacles amovibles (10A, 10B, 10C, 10A′, 10B′, 10C′; 100A, 100B, 100C, 100A′, 100B′, 100C′; 1000A, 1000B, 1000C, 1000A′, 1000B′, 1000C′) de manière que les rangées (32, 34; 320, 340; 3200, 3400) puissent être de longueur variable.

3. Appareil de test (10A, 10B, 10C, 10A′, 10B′, 10C′, 30; 100A, 100B, 100C, 100A′, 100B′, 100C′, 300; 1000A, 1000B, 1000C, 1000A′, 1000B′, 1000C′, 3000) pour conduire des immunoessais comprenant plusieurs puits de test (17, 18, 19, 17′, 18′, 19′) et à chacun une surface inférieure construite pour recevoir tout antigène ou anticorps approprié pouvant être présent dans un échantillon et pour former un complexe antigène-anticorps,
caractérisé en ce que les puits (17, 18, 19, 17′, 18′, 19′) sont montés dans au moins deux rangées généralement adjacentes et parallèles (32, 34; 320, 340; 3200, 3400), une rangée (34; 340; 3400) étant construite pour recevoir uniquement les matières qui ne produisent pas de changement de couleur visible dû à l'antigène ou l'anticorps recherché, comme témoin négatif, et la rangée (32; 320; 3200) adjacente à la rangée (34; 340; 3400) étant construite pour recevoir que les essais qui peuvent produire un changement de couleur, sous la forme soit d'un échantillon de malade contenant l'antigène ou anticorps suspecté, ou d'un témoin positif contenant un antigène ou anticorps qui produit un changement de couleur afin d'identifier facilement un échantillon de malade qui a l'antigène ou l'anticorps par comparaison de chaque puits portant une couleur (17, 18, 19) d'une rangée (32; 320; 3200) avec un puits ne portant pas de couleur (17′, 18′, 19′) de la rangée adjacente (34; 340; 3400).

4. Nécessaire expérimental (10A, 10B, 10C, 10A′, 10B′, 10C′, 30; 100A, 100B, 100C, 100A′, 100B′, 100C′, 300; 1000A, 1000B, 1000C, 1000A′, 1000B′, 1000C′, 3000) pour conduire des immunoessais comprenant un cadre (30; 300; 3000) et plusieurs réceptacles (10A, 10B, 10C, 10A′, 10B′, 10C′; 100A, 100B, 100C, 100A′, 100B′, 100C′; 1000A, 1000B, 1000C, 1000A′, 1000B′, 1000C′) montés de manière amovible dans le cadre (30; 300; 3000), chacun des réceptacles (10A, 10B, 10C, 10A′, 10B′, 10C′; 100A, 100B, 100C, 100A′, 100B′, 100C′; 1000A, 1000B, 1000C, 1000A′, 1000B′, 1000C′) ayant plusieurs puits de test (17, 18, 19; 17′, 18′, 19′) positionnés pour former au moins deux rangées (32, 34; 320, 340; 3200, 3400) avec au moins deux des puits (17, 18, 19, 17′, 18′, 19′) dans chaque rangée (32, 34; 320, 340; 3200, 3400), les puits (17′, 18′, 19′) d'une des rangées (34; 340; 3400) étant construits et conçus pour ne recevoir que les matières pour le témoin négatif et les puits (17, 18, 19) de l'autre rangée (32; 320; 3200) étant construits sous forme de puits producteurs de couleur qui sont conçus pour ne recevoir que des matières pour témoin positif ou d'échantillon de malade de manière qu'aucun puits producteur de couleur ne soit positionné entre un puits (17, 18, 19) pour le liquide d'échantillon de malade et un puits témoin négatif (17′, 18′, 19′).

5. Procédé pour conduire l'immunoessai pour un antigène ou anticorps ciblé dans plusieurs échantillons de malade utilisant plusieurs puits de test (17, 18, 19, 17′, 18′, 19′) et un témoin positif et négatif, les témoins comprenant les réactifs qui, s'ils sont convenablement combinés et maintenus sous forme active, forment toujours un changement de couleur substantiel dans le cas du témoin positif et ne forment jamais un changement de couleur substantiel dans le cas du témoin négatif en l'absence de l'antigène ou de l'anticorps recherché, le procédé comprenant les étapes de:
a) disposition de puits de test (17, 18, 19, 17′, 18′, 19′) en deux rangées (32, 34; 320, 340; 3200, 3400), chaque puits (17, 18, 19, 17′, 18′, 19′) dans une rangée étant adjacents à un puits de l'autre rangée;
b) dépôt dans les puits (17′, 18′, 19′) d'une des rangées (34, 340; 3400) uniquement des matières du témoin négatif dans la séquence appropriée qui ne produit pas de changement de couleur substantiel;
c) dépôt dans les puits respectifs (17, 18, 19) de l'autre des rangées (32; 320; 3200) soit d'échantillons de malade soit de certains témoins positifs, et des réactifs nécessaires pour faire le test de l'antigène ou anticorps ciblé; et
d) comparaison du défaut de changement de couleur substantiel dans les puits (17′, 18′, 19′) d'une rangée (34; 340; 3400) avec la présence ou l'absence d'un changement de couleur substantiel dans un puits (17, 18, 19) de l'autre rangée (32; 320; 3200) qui lui est adjacente, pour déterminer que l'antigène ou anticorps ciblé est présent ou absent dans les puits d'échantillon de malade (17, 18, 19).
